(19) Europäisches Patentamt
European Patent Office
Office européen des brevets

(11) **EP 1 713 481 B1**

(12) **EUROPEAN PATENT SPECIFICATION**

(45) Date of publication and mention
of the grant of the patent:
**12.06.2013 Bulletin 2013/24**

(51) Int Cl.:
*A61K 31/4439* (2006.01)   *A61P 35/00* (2006.01)
*A61P 31/16* (2006.01)   *A61P 19/02* (2006.01)

(21) Application number: **05707696.0**

(22) Date of filing: **14.02.2005**

(86) International application number:
**PCT/EP2005/002250**

(87) International publication number:
**WO 2005/077365 (25.08.2005 Gazette 2005/34)**

(54) **NOVEL USES FOR PROTON PUMP INHIBITORS**

NEUE ANWENDUNGEN FÜR PROTONENPUMPENINHIBITOREN

UTILISATIONS D'INHIBITEURS DE POMPE A PROTONS

(84) Designated Contracting States:
**AT BE BG CH CY CZ DE DK EE ES FI FR GB GR
HU IE IS IT LI LT LU MC NL PL PT RO SE SI SK TR**

(30) Priority: **12.02.2004 GB 0403165**

(43) Date of publication of application:
**25.10.2006 Bulletin 2006/43**

(73) Proprietor: **ISTITUTO SUPERIORE DI SANITA'
00161 Roma (IT)**

(72) Inventors:
• **FAIS, Stefano**
  **I-Roma (IT)**
• **LUCIANI, Francesca**
  **I-Roma (IT)**

(74) Representative: **Lord, Hilton David
Marks & Clerk LLP
90 Long Acre
London
WC2E 9RA (GB)**

(56) References cited:
EP-A- 0 567 643    EP-A- 1 306 375
WO-A-02/13796    WO-A-89/03829
WO-A-97/40039    WO-A-02/058697
WO-A-02/080917    WO-A-02/080917
WO-A-2004/006849    WO-A-2004/064719
WO-A-2004/073654    WO-A-2005/051432
WO-A-2006/021456    WO-A1-01/44257
WO-A2-2004/006849    GB-A- 2 336 311
US-A- 5 693 818    US-A- 6 015 801
US-B1- 6 489 346

• **SALEH M N ET AL: "Phase I trial of the anti-Lewis
Y drug immunoconjugate BR96-doxorubicin in
patients with lewis Y-expressing epithelial
tumors." JOURNAL OF CLINICAL ONCOLOGY :
OFFICIAL JOURNAL OF THE AMERICAN
SOCIETY OF CLINICAL ONCOLOGY JUN 2000,
vol. 18, no. 11, June 2000 (2000-06), pages
2282-2292, XP002460051 ISSN: 0732-183X**
• **SARTORI S ET AL: "Randomized trial of
omeprazole or ranitidine versus placebo in the
prevention of chemotherapy-induced
gastroduodenal injury." JOURNAL OF CLINICAL
ONCOLOGY : OFFICIAL JOURNAL OF THE
AMERICAN SOCIETY OF CLINICAL ONCOLOGY
FEB 2000, vol. 18, no. 3, February 2000 (2000-02),
pages 463-467, XP002460052 ISSN: 0732-183X**
• **DATABASE EMBASE [Online] ELSEVIER
SCIENCE PUBLISHERS, AMSTERDAM, NL; 1
September 2002 (2002-09-01), STEER C B ET AL:
"Gastro-oesophageal complications in patients
receiving cancer therapy: The role of proton
pump inhibitors" XP002460055 Database
accession no. EMB-2002371421 & EUROPEAN
JOURNAL OF GASTROENTEROLOGY AND
HEPATOLOGY 01 SEP 2002 UNITED KINGDOM,
vol. 14, no. SUPPL. 1, 1 September 2002
(2002-09-01), pages S17-S21, ISSN: 0954-691X**
• **LUCIANI FRANCESCA ET AL: "Effect of proton
pump inhibitor pretreatment on resistance of
solid tumors to cytotoxic drugs." JOURNAL OF
THE NATIONAL CANCER INSTITUTE 17 NOV
2004, vol. 96, no. 22, 17 November 2004
(2004-11-17), pages 1702-1713, XP002460053
ISSN: 1460-2105**

EP 1 713 481 B1

**(Cont. next page)**

- EUROPEAN MEDICINES AGENCY: "European Public Assessment Report (EPAR) PHOTOBARR"[Online] 2007, XP002460054 Retrieved from the Internet: URL:http://www.emea.europa.eu/humandocs/PD Fs/ EPAR/PhotoBarr/082204en1.pdf> [retrieved on 2007-11-26]
- DATABASE WPI Section Ch, Week 200335 Derwent Publications Ltd., London, GB; Class B02, AN 2003-371904 XP002329118 & WO 03/027098 A1 (TAKEDA CHEM IND LTD) 3 April 2003 (2003-04-03)
- DATABASE WPI Section Ch, Week 200382 Derwent Publications Ltd., London, GB; Class B02, AN 2003-884852 XP002329119 & JP 2003 277262 A (TAKADA K) 2 October 2003 (2003-10-02) cited in the application
- DATABASE WPI Section Ch, Week 200410 Derwent Publications Ltd., London, GB; Class B02, AN 2004-095264 XP002329120 & KR 2003 072 705 A (KOLON CHEM CO LTD) 19 September 2003 (2003-09-19)
- GRAHAM D Y: "Pancreatic enzyme replacement: the effect of antacids or cimetidine" DIGESTIVE DISEASES AND SCIENCES, PLENUM PUBLISHING CO, US, vol. 27, no. 6, June 1982 (1982-06), pages 485-490, XP002085957 ISSN: 0163-2116
- RAYBURN W ET AL: "ANTACIDS VS. ANTACIDS PLUS NON-PRESCRIPTION RANITIDINE FOR HEARTBURN DURING PREGNANCY" INTERNATIONAL JOURNAL OF GYNECOLOGY AND OBSTETRICS, NEW YORK, NY, US, vol. 66, no. 1, 1999, pages 35-37, XP001122187 ISSN: 0020-7292

Remarks:

The file contains technical information submitted after the application was filed and not included in this specification

**Description**

The present invention provides the use of proton pump inhibitors, such as omeprazole, in antineoplastic therapies.

Existing antineoplastic strategies have shown low levels of efficacy against solid tumours, together with high *in vivo* systemic toxicity, and there remains a need for new antitumour strategies with low systemic toxicity.

Our recent data suggest that tumour malignancy and invasiveness are associated with two main mechanisms: (i) an aberrant phagocytic activity (Lugini *et al.,* 2003); and, (ii) the release of exosomes able to kill lymphocytes through a Fas-mediated apoptotic mechanism (Andreola *et al.,* 2002). It is conceivable that a common mechanism links the two, possibly involving the traffic of strongly acidified vesicles, belonging to a powerful lysosomal network. Inhibition of the actin cytoskeleton connection to the lysosomal membranes may impair these tumour functions.

The influence of pHi (intracellular pH) has been studied with respect to cell growth, cell motility, tumorigenesis, metastasis and apoptosis in cancer cells (Perona *et al.,* 1988; Schlappack *et al.,* 1991; Gottlieb *et al,* 1995; Helmlinger *et al.,* 1997; Martinez-Zaguilan *et al.,* 1998).

The microenvironment of solid tumours contains regions of poor oxygenation and high acidity. Growing evidence from clinical and experimental studies points to a fundamental role of an acidic tumour microenvironment in metastatic progression (Subarsky and Hill, 2003). Decreased $pO_2$, acidity, and the lack of nutrients alter gene expression. Genes that play a role in angiogenesis, tissue remodelling, and survival, are necessary for the survival of tumour cells, and play a pivotal role in metastatic progression (Subarsky and Hill, 2003).

The extracellular (interstitial) pH of solid tumours is significantly more acidic than that of normal tissues (Izumi H *et al.,* 2003). Although the data are limited, pH measurements in humans indicate a difference between tumour and normal tissues (Tannock and Rotin 1989). Moreover, acidic intracellular organelles can also participate in resistance to chemotherapeutic drugs (Altan *et al.,* 1998; Hurwitz *et al.,* 1997; Schindler *et al.,* 1996; Larsen *et al.,* 2000; Raghunand *et al.,* 1999; Ouar *et al.,* 1999), thus conferring to tumour cells a cumulative overall selective advantage.

The acidity displayed by tumours has been proposed as a potentially useful tool to distinguish tumour tissues from healthy ones. Possible tumour functions, in which the acidification of extracellular environment and lysosomal compartments may have a role, include: (i) direct impairment of lymphocyte functions through acidity (Ratner and Heppner, 1985); and, (ii) the raising of a chemical/physical barrier capable to inactivate and/or sequestrate chemotherapeutic drugs (Altan *et al.,* 1998).

It is conceivable that the acidic tumour microenvironment, and the highly efficient tumour lysosomal compartment, of tumour cells, together may represent a sort of digestive apparatus, allowing tumour cells to feed by way of an extracellular matrix on dead cells (Lugini *et al.,* 2003). Thus, it may be that tumour microenvironment acidification presents as an overall selective advantage.

Enhanced expression and activity of Vacuolar type $H^+$ ATPases (VH$^+$ ATPases), a class of active $H^+$ transporters, in tumour cells, may have a key role in the acidification both of the tumour extracellular microenvironment and of the intracellular acidic compartments.

The acidic microenvironment of cancer cells has also been associated with multidrug resistance. Resistance to chemotherapeutic agents is a major cause of treatment failure in patients with cancer, and can be caused by biochemical and/or physiological mechanisms. Biochemical mechanisms include the over-expression of resistance-conferring proteins, such as, for example, P-glycoprotein (P-gp), a plasma-membrane drug efflux transporter. Physiological resistance involves the tumour microenvironment, and can be caused by alterations of intra- and/or extra-cellular pH.

*In vitro,* low pH reduces the uptake of weakly basic chemotherapeutic drugs and, hence, reduces their cytotoxicity (Raghunand *et al.* 1999). This phenomenon has been postulated to contribute to a 'physiological' resistance to weakly basic drugs *in vivo,* and data obtained in animal models show that bicarbonate-induced extracellular alkalinisation leads to significant improvements in the therapeutic effectiveness of some chemotherapeutic drugs, including doxorubicin, against human tumour cells, both *in vitro* and *in vivo* (Raghunand *et al.* 2003; Mahoney *et al.* 2003). Studies in model systems have demonstrated that tumour pH can be a determinant of treatment response.

Acidic intracellular organelles can also participate in resistance to chemotherapeutic drugs (Altan *et al.,* 1998; Hurwitz *et al.,* 1997; Schindler *et al.,* 1996; Larsen *et al.,* 2000; Raghunand *et al.,* 1999; Ouar *et al.,* 1999). The turnover of acidic vesicles may represent an important factor in chemoresistance, especially in cells that do not over-express plasma membrane bound drug pumps, such as P-glycoprotein. In fact, some data suggest that chemotherapeutic drugs distribute through the cytoplasm and nucleoplasm of drug-sensitive cells, but are excluded from the nucleus in drug-resistant cells (Altan *et al.,* 1998; Hurwitz *et al.,* 1997; Schindler *et al.,* 1996; Larsen *et al.,* 2000; Raghunand *et al.,* 1999; Ouar *et al.,* 1999). Indeed, several reports suggest that increased acidification of lysosomal-type vesicles is causally related to drug resistance and is consistent with the hypothesis that sequestration of drugs in acidic organelles and subsequent extrusion from the cell through the secretory pathway contribute to chemotherapeutic resistance (Schindler *et al.,* 1996; Hurwitz *et al.,* 1997; Cleary *et al.,* 1997; Altan *et al.,* 1998; Raghunand *et al.,* 1999; Ouar *et al.,* 1999; Bour-Dill *et al.,* 2000; Larsen *et al.,* 2000). Moreover, some recent findings indicate that lysosomotropic agents that impair the acidic-pH-dependent accumulation of weak-base chemotherapeutic drugs may reverse anthracycline resistance in MDR cells with an expanded acidic lysosomal compartment (Ouar *et al.,* 2003).

Vacuolar H$^+$-ATPases (V-H$^+$-ATPases) are a class of transporters involved in the control of pH in many cellular compartments. This family of efflux pumps has a number of functions in eukaryotic organisms, and is diffusely expressed in many cellular types, including some human tumour cells (Beck, 1987; Vaananen *et al.,* 1990; Marquardt *et al.,* 1991; Martinez-Zaguilan, 1993; Moriyama, 1996; Murakami *et al.,* 2001). These ATPases carry out ATP-dependent proton transport from the cytoplasmic compartment to the opposite side of the membrane, that in turn may be represented by either the lumen of an intracellular organelle or the extracellular space.

Proton pump inhibitors (PPIs), including omeprazole and its analogues, such as esomeprazole, lansoprazole, pantoprazole, and rabeprazole, specifically inhibit pumps responsible for the active transport of H$^+$ ions from cytoplasm across the plasma membrane (to the extracellular space) or across the vacuolar membrane (to the lumen of acidic vesicles). These molecules are drugs currently used to treat the symptoms of peptic disease.

JP 2003277262 discloses a combination of lansoprazole and a mixture of esters for use as an anticancer agent in the gut, wherein the esters increase the bioavailability of lansoprazole.

JP 2001286284 discloses a peptide sub unit of V-ATPase and antibodies thereagainst having PPI activity. It is speculated that these antibodies may have anticancer activity, as V-ATPase is associated with tumours.

WO 02/080917 discloses the use of proton pump inhibitors for the treatment of induced multi-drug resistance (MDR) in conditions including malaria and cancer. Simultaneous administration of the proton pump inhibitor and anti-cancer agent is suggested. Induced MDR is associated with characteristic protein expression, such as protein transporters, including the ABC protein transporters. Inherent resistance, which is not associated with such expression, is not addressed, and is a considerably more serious problem in cancer therapy. The authors report that simultaneous treatment with the PPI and a drug, such as doxorubicin or vincristine, apparently led to a small increase in drug sensitivity of the tumour cells.

EP 0567643 discloses a number of novel anti-ulcer agents, and the authors speculate that these agents may also possess anticancer activity, although there are no data to support this.

EP 1 306 375 discloses salts of lansoprazole as well as possible uses in the treatments of gastric conditions.

WO 89 103 829 discloses the treatment of osteoporosis and tumour associated hypercalcemia.

Surprisingly, we have now found that the proton pump inhibitors, on their own, are able to exert antineoplastic effects on solid tumours, and that they are able to substantially completely restore drug sensitivity to such tumours, where resistance is presented, when used as a pre-treatment.

Thus, in a first aspect, the present invention provides a 2-pyridyl methylsulphinyl benzimidazole proton pump inhibitor for use in the systemic, therapeutic treatment of a solid tumour by oral administration.

It is further preferred that the tumour is either metastatic, or that there is a significant chance that the tumour is or will be metastatic, as diagnosed by a skilled physician, for example.

As noted above, it is particularly metastatic tumours that are associated with acidic conditions, both intra- and extra-cellularly, and it has been found that, surprisingly, omeprazole and other PPIs are able to exert a systemic effect on such tumours.

It is advantageous to be able to assess the pH of a tumour before and after administration of the PPI, as this can assist the skilled physician in determining both the amount and the type of PPI best suited to treat the individual patient. Such monitoring also provides for the assaying, *in vivo,* of the direct effect of PPI treatment on tumour pH.

Indeed, in the accompanying Examples, we provide the results from *in vitro* experiments using human cell lines derived from tumours of varying histology, and *in vivo* experiments grafting the same tumor cells into Severe Combined Immunodeficiency (SCID) mice (Lozupone *et al.,* 2000; 2003; 2004 in press), and show that: (i) omeprazole is cytotoxic in a dose-dependent manner, for tumour cells cultured in slightly acidic medium; (ii) it is non-toxic for the same tumour cells cultured in buffered medium; (iii) *in vivo* treatment of human-SCID with omeprazole or its analogues was able to markedly reduce tumour growth, and (iv) that pre-treatment with omeprazole was generally able to reverse, often substantially completely, multidrug resistance (MDR).

PPIs display an important chemical feature in that, being weak bases, they accumulate in acidic compartments, and are activated through protonation, exerting their function as proton pump inhibitors. Thus, they normally accumulate in the stomach.

Proton pump inhibitors (PPIs) have emerged as the drug class of choice for treating patients with acid-related diseases, including gastro-oesophageal reflux disease, duodenal and gastric ulcers. These agents inhibit gastric acid secretion by targeting the gastric acid pump (Larsson *et al.,* 1985; Wallmark *et al.,* 1985; Puscas *et al.,* 1999; Horn 2000). PPIs (typically omeprazole, lansoprazole, pantoprazole, and rabeprazole) are substituted 2-pyridyl methylsulphinyl benzimidazoles that share a similar core structure (Horn 2000). These agents are protonable weak bases with pK$_a$ values of ~4 and, so, accumulate selectively in acidic spaces with a pH of <4. In such an acidic environment, protonation of the pyridine and benzimidazole nitrogens results in the formation of a tetracyclic sulphenamide, which represents the active form of the drug (Horn 2000).

Preferred 2-pyridyl methylsulphinyl benzimidazole PPI's, are omeprazole, lansoprazole, pantoprazole, and rabeprazole.

In one aspect, it is preferred to treat a cancerous condition that is not a gut cancer. In another aspect, it is preferred not to use lansoprazole, especially where the cancerous condition is associated with the gut.

Although tumour cells are able to survive in neutral, buffered media, we have surprisingly found that when PPIs are used to block the pH fluctuations (lower extracellular, lower intra-vacuolar pH and higher cytoplasmic pH) induced by vacuolar-type $H^+$-ATPase expression and activity in tumour cells, these tumours can be controlled and even killed.

It is particularly surprising is that the PPIs are able to exert a systemic effect on tumours, despite being expected to be bound, or sequestered, in the stomach on ingestion. Nevertheless, it is preferred to administer the PPI in conjunction with an antacid, for example, in order that any acidic stomach condition need not reduce the effectiveness of any orally administered PPI. Thus, it is preferred to administer sufficient antacid to the patient to facilitate uptake of the PPI. Such a dose may be any that is readily determined by the skilled physician but may, as a guide, be that amount recommended by the manufacturer for treating acid reflux, for example.

It will be appreciated that where omeprazole, or PPI, are mentioned herein, then either term relates to any PPI useful in the present invention, unless otherwise apparent. Likewise, where reference is had to the term "tumour", this refers to a solid tumour of the invention.

The PPI may be administered in any amount effective to exert an antineoplastic effect. In general, this may be in about the same amount as used for the treatment of a stomach ulcer, for example. An acceptable dosage for omeprazole is generally between 20-40 mg/day. This dosage may increase for other analogues - for example, pantoprazole may typically be administered at 40-80 mg/day. However, even overdoses of 560 or 2400 mg/day have shown neither considerable nor stable side effects.

It will be appreciated that the amounts of PPI used may vary with the patient and their condition, as will be apparent to the skilled physician, and may vary typically upwards, especially if the patient has a peptic condition, for example, that is not being treated by an antacid, for instance. The PPI may suitably be administered in conventional form, as provided by the manufacturer, such forms particularly including any suitable for oral administration, including tablets, capsules and lozenges, for example, as well as delayed and sustained release formulations.

It is generally preferred to pre-treat the tumour patient with another antacid, such as calcium carbonate, or antacid drug, such as an $H_2$-receptor antagonist, for example ranitidine or cimetidine, in order to inhibit acid secretion in the stomach, thereby enhancing the concentration of PPI able to reach the tumour site, and minimising the concentration remaining in the stomach. Thus, treatment with an antacid is effective to increase the delivery of PPI to the acidic tumour, as the number of only acidic sites in the body is substantially reduced or, at least, the most significant site is temporarily neutralised, or ameliorated.

In general, the PPI drugs appear to be equally effective in treatment of solid tumours. Particularly preferred are omeprazole, esomeprazole, lansoprazole, pantoprazole, and rabeprazole, with omeprazole being more preferred. Any medicament may contain one or more PPI as the active ingredient.

PPIs have been found to be surprisingly effective in the treatment of tumours that have become, or are, intractable to other drugs. In particular, we have found that tumours that have the MDR phenotype lose their drug resistance, or resistance is significantly diminished, when pretreated with a PPI. Such pretreatment appears to block the acidifying effect of the resistant tumour, which also appears to lift drug the resistance, leaving the tumour exposed to the effect of the drug selected for its treatment. This result was entirely unexpected.

What is especially surprising is that the effect does not appear to be limited to lightly basic drugs, but appears to lift resistance across the spectrum, in all tested drugs, and restores efficacy, regardless of drug type. What also appears to be the case is that contemporaneous treatment, with no prior treatment with PPI, has no effect, or very little useful effect, and that the tumour needs to be pretreated in order to restore sensitivity.

The reason for the lack of efficacy of the PPI when administered simultaneously with another anticancer drug, such as cisplatin, is unclear, but is clearly demonstrated in the accompanying Examples. Pretreatment yields good results, but results with no pretreatment and only concurrent PPI treatment are comparable to the control with no PPI present. Without being restricted by theory, it appears possible that a drug, such as cisplatin, is having the opposite effect from the PPI, and is activating the acidifying ATPase, thereby neutralising the effect of the PPI, if the PPI has not had the opportunity to have an effect. It is also possible that the two drugs are competing for sequestration in the acidic tumour environment, given that the great majority of anti-cancer drugs are weak bases and that, like the PPI's, require protonation in order to be activated.

Thus, in joint therapy of a cancer, where a PPI is used in conjunction with a second or further anticancer drug, the patient should be pretreated with the PPI, prior to administration of the second or further anticancer drug, which may be a standard cancer treatment. This treatment with PPI may be one, or a series, of treatments, or a continuous treatment, such as by catheter or a transdermal patch, over the previous day or so, by any suitable means, such as oral, systemic or local administration. The length of time is not especially important, provided that the PPI has had a chance to act, and that the effect of the PPI is still present when the other anticancer drug is administered, insofar as the acidic environment is still at least partially compromised at the time of subsequent treatment.

Thus, in a further aspect, the present invention provides the use of a proton pump inhibitor in the manufacture of a medicament for the joint treatment of a cancerous condition, wherein the proton pump inhibitor medicament is for administration prior to the joint therapy.

In such use, it is particularly preferred that the administration be sufficiently prior to the joint therapy as to reduce the acidity associated with the site of the said condition. The period prior to administration of the joint therapy is preferably between 30 minutes and 3 days. Preferably, the PPI is administered on one or more occasions prior to administration of the joint therapy, the first being at least one day prior to therapy, and preferably followed by another between 2 and 12 hours prior to the joint therapy.

A pretreatment period of about 24 hours may be appropriate, but it is preferred that the PPI be administered at least an hour before treatment, and preferably at least two or more hours, in order to allow the maximum effect of the PPI. The treatment need not necessarily be continuous, once started, as it has been found that the PPI effects on tumour acidity last for a small number of days, such as two or three. However, as PPI treatment is safe, it is generally preferred simply to continue the treatment.

The PPI may be administered continuously over the course of the treatment with the other drug, but it is preferred to administer it as part of a regimen, where a treatment with the PPI is given for a period of between, say, 6 and 24 hours before the other drug, followed by administration of the other drug, with the cycle being repeated as appropriate to the drug to which the tumour would otherwise be resistant. Where this would normally be administered on a daily basis, the PPI may be administered effectively on a continuous basis, or, say, 6 hours before the drug.

Examples of other drugs, against which resistance can be overcome by PPIs, include: those where MDR appears to be related to efflux pump proteins, including; vinka alkaloids, such as vinblastin, vincristine, vinorelbine, and vindesine; taxanes, such as paclitaxel, and docetaxel; anthracyclines, such as doxorubicin, daunorubicin, epirubicin, and idarubicin; anthracenes, such as bisanthrene, and mitoxanthrene; epipodophyllotoxins, such as etoposide, and teniposide; camptothecins, such as topotecan, and irinotecan/sn38; heavy metal oxyanions, such as arsenite, and trivalent antimony; actinomycin d; mitomycin c; methotrexate; trimetrexate; amsacrine; imitinib; and melphalan; and those where MDR appears not to be related with protein-mediated efflux pumps, including 5-fluorouracil (5-fu) and cisplatin.

It will be appreciated that, while the PPI's possess anticancer activity, when used as part of a joint therapy, it is their ability to reduce drug resistance to another anticancer drug that will generally be of importance, although any cumulative anticancer effect can only be of benefit.

Thus, in an alternative aspect, the present invention provides the use of a PPI in the preparation of a medicament for the treatment of a solid tumour resistant to one or more antineoplastic drugs in a patient having sub-effective levels of a drug to which the condition is resistant. In such a case, a patient may be on a course of drugs for a cancerous condition, and administration of the PPI is timed such as to have an effect on the acidic microenvironment of the tumour when levels of the other anticancer drug have dropped sufficiently to permit the PPI to have such an effect. Given the toxic effects of most anticancer drugs, it is conventional to allow the patient to recuperate between administrations, so that windows of opportunity are readily found and established by those skilled in the art. That PPI's continue to have the desired effect after 2 or 3 days is also of assistance, so that a patient may readily take the appropriate amount of PPI on the day before the next course of anticancer drug.

Thus, in this context, "sub-effective" indicates that the level of drug is not sufficient to prevent the MDR-reducing effect of the PPI at the time of administration, or at a subsequent stage when sufficient PPI is systemically effective. Preferably, the level of drug to which the condition is resistant should be allowed to drop to negligible levels for at least a short period, during which the PPI is preferably administered and can act.

The present invention is also useful in the long term therapy and treatment of solid tumour. For example, tamoxifen is used both to prevent breast cancer in women thought to be at high risk, as well as in the ongoing treatment of individuals who have overcome breast cancer, in order to prevent a recurrence. In particular, there is evidence that tamoxifen increases intracellular pH.

The risk of developing resistance is increased in individuals subject to ongoing treatment, so that concurrent administration of a PPI, for example with PPI being administered on days when the drug, such as tamoxifen, is not, can help either to prevent resistance from developing, or help to stop resistance hindering ongoing treatment.

Tamoxifen has a high level of systemic toxicity, while the PPI's have been established to be non-toxic, even in overdosage regimens. Thus, in accordance with the present invention, PPI's may either be used as an alternative drug to tamoxifen, or combined with tamoxifen in the prevention of cancer relapses.

Likewise, other forms of hereditary cancer treated on a prophylactic basis are also susceptible to such treatment. An example is colonic polyposis, and other conditions which environmental factors may encourage.

The present invention further provides a combined therapy for the treatment of a disease condition, said combined therapy comprising administration of

a) a proton pump inhibitor, and

b) at least one drug, other than a proton pump inhibitor, for the treatment of said condition, to a patient in need thereof, and wherein said proton pump inhibitor is administered to said patient prior to said at least one drug.

Administration of the PPI is preferably at a time sufficiently prior to the second drug to allow the PPI to at least partially neutralise any acidic microenvironment associated with the condition. More preferably, where the second drug is administered as part of an ongoing regimen, said second drug should be at a concentration below that deemed to be effective for the condition. Thus, it is preferred that the second drug not be administered by patch or, if it is, then the patch should be removed to allow levels of drug to drop below the effective.

It will be appreciated that such regimens may be followed in continuing therapy to prevent recurrence of a condition, as well as to treat the condition, and may also be employed in the prevention of a condition, particularly in high-risk individuals.

While it has not been confirmed, it seems possible that pH alterations in tumours present an overall selective advantage, rendering tumour cells able to survive, block immune response and spread. Drugs able to act through inhibition of proton pumps and, thus, regulate cellular pH, appear to be able to hinder the advantage represented by pH alterations. This advantage may be conferred by the V-H$^+$ ATPases and, indeed, we have found that specific inhibitors of these enzymes, including omeprazole, and its analogues lansoprazole, rabeprazole and pantoprazole, have potent anti-tumour activity.

**Brief Description of the Drawings**

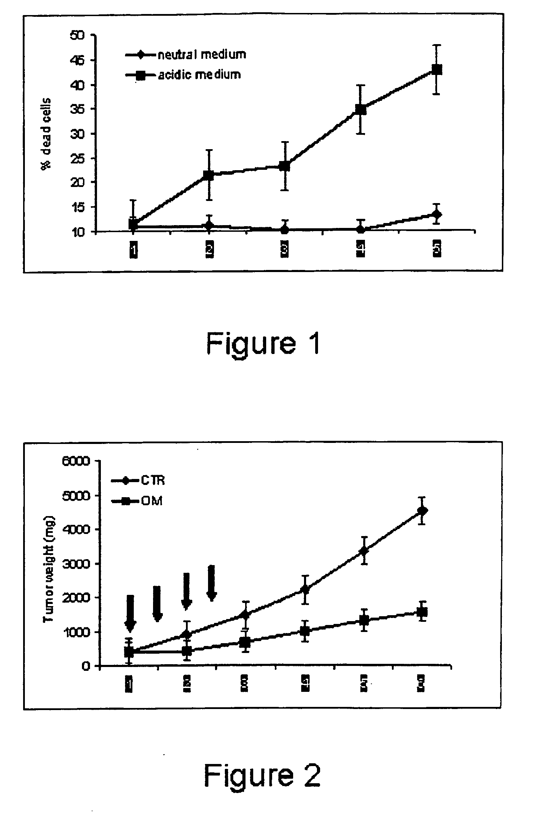

**Figure 1: Cytotoxic effect of omeprazole *in vitro***

The figure shows a dose response curve obtained treating human melanoma cells with 4 logarithmic dilution (x axis: 0 $\mu$g/ml, 0.01 $\mu$g/ml, 0.1 $\mu$g/ml, 1 $\mu$g/ml, 10 $\mu$g/ml) of omeprazole, in buffered ($\blacklozenge$, neutral, pH7,2) or not buffered ($\blacksquare$, slightly acidic) culture medium. The results clearly showed that omeprazole was cytotoxic in a dose-dependent manner only for tumour cells grown in slightly acidic medium. Figure shows the median results from three independent experiments. Error bars = standard deviation.

**Figure 2: Effects of omeprazole on human tumour growth *in vivo***

The figure shows the median results of three representative *in vivo* experiment on CB.17 *scid/scid* mice engrafted with a human melanoma cell line, derived from a primary lesion. Mice engrafted with tumour cells were treated ($\blacksquare$), four times as indicated by arrows) or not ($\blacklozenge$) with omeprazole by gavage. Tumour weight (mg) is represented as a function of time. To note the straightforward effect of omeprazole in inhibiting tumour growth. Error bars = standard deviation.

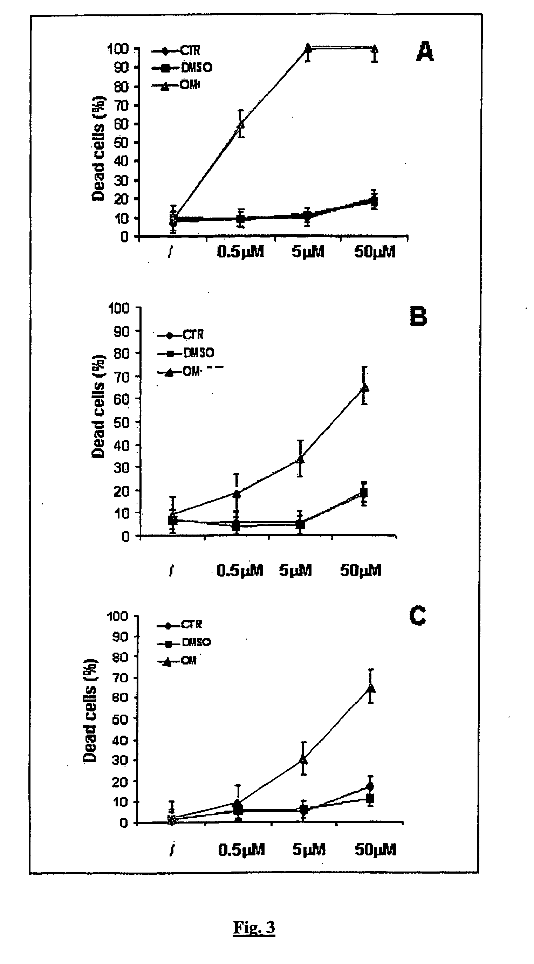

**Figure 3: Effects of omeprazole on cisplatin resistance**

The Figure shows three (A,B,C) representative dose response curves obtained treating human melanoma cells with three logarithmic dilution (as indicated on X-axes) of cisplatin alone (CTR line) or after a 24 hrs pre-treatment with omeprazole (OM line). As a control (DMSO line) cells were treated with cisplatin plus DMSO, being DMSO the medium in which omeprazole was solubilised in stock solution. The DMSO final concentration was the same resulting from omeprazole treatment of cells, namely 0.0008%. The results clearly showed that omeprazole was able to almost completely restored cell sensitivity to cisplatin (OM line) on human melanoma cells resistant to cisplatin treatment (CTR line).

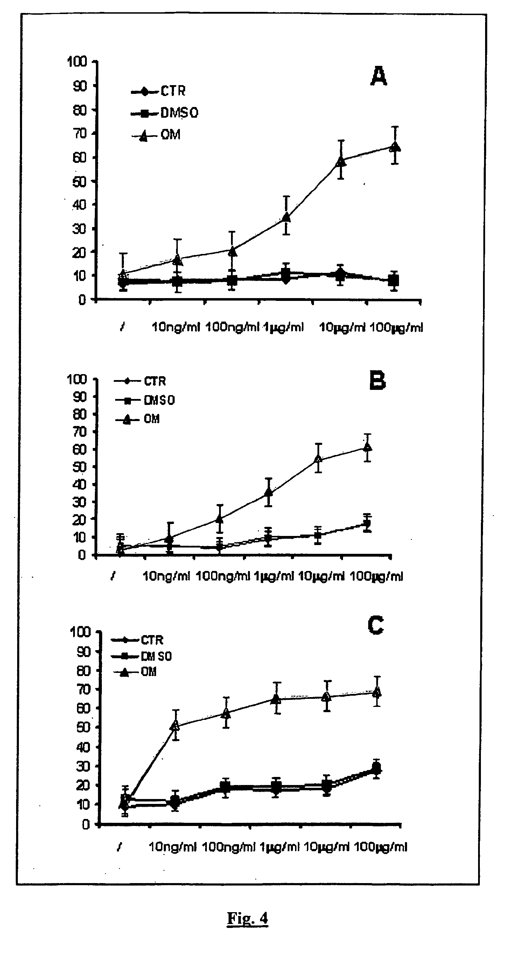

**Figure 4: Effects of omeprazole on 5-Fluorouracil (5-FU) resistance**

The Figure shows three representative dose response curves obtained treating human colon adenocarcinoma (A) or melanoma (B, C) cells with five logarithmic dilution (as indicated on the X-axes) of 5-fluorouracil (5-FU) alone (CTR line) or after 24 hrs pre-treatment with omeprazole (OM line). As a control (DMSO line) cells were treated with 5-FU plus DMSO, being DMSO the medium in which omeprazole was solubilised in stock solution. The DMSO final concentration was the same resulting from omeprazole treatment of cells, namely 0.0008%. The results clearly showed that omeprazole was able to almost completely restored cell sensitivity to 5-FU (OM line) on human tumour cell lines resistant to cisplatin treatment (CTR line).

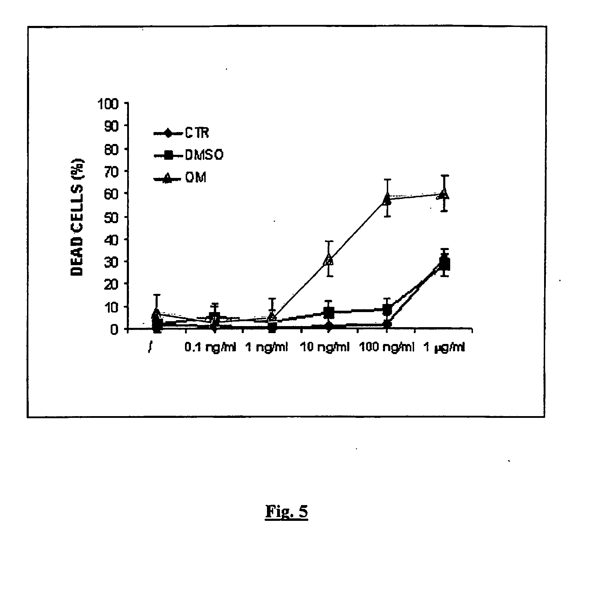

**Figure 5: Effects of omeprazole on P-gp expressing multidrug resistant cells**

The Figure shows one representative dose response curves obtained treating CEM-VBL100 cell line with five logarithmic dilution (as indicated on X-axes) of vinblastine sulphate (VBL) alone (CTR line) or after 24 hrs pre-treatment with omeprazole (OM line). As a control (DMSO line) cells were treated with VBL plus DMSO, being DMSO the medium in which omeprazole was solubilised in stock solution. The DMSO final concentration was the same resulting from omeprazole treatment of cells, namely 0.0008%. The results clearly showed that omeprazole was able to almost completely restored cell sensitivity to VBL (OM line) on human tumour cell lines resistant to cisplatin treatment (CTR line).

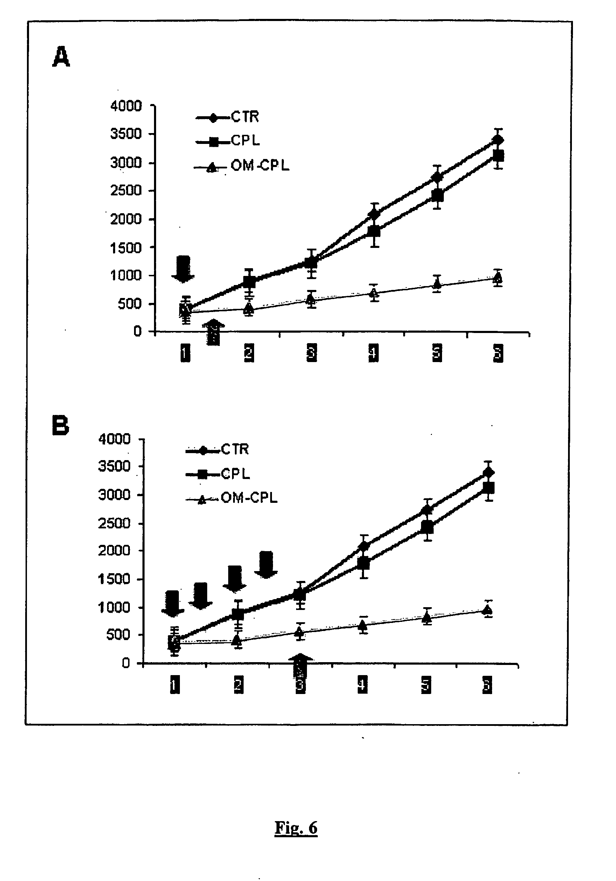

**Figure 6: *In vivo* effects of omeprazole on tumour growth in the human/SCID mouse model**
SCID mice were engrafted with a melanoma cell line via s.c. injection into the right flank. At the tumour appearance, mice were left untreated (CTR line) or were treated with omeprazole (1 single gavage treatment at day 1, heavy, down arrow) and cisplatin (1 single i.p. treatment at day 2, lighter, up arrow) OM-CPL line or cisplatin alone (CPL line). Graphs represent tumour growth expressed as mg weight (see Materials and Methods) as a function of time. Results clearly showed that omeprazole strongly enhanced tumour sensitivity to cisplatin (OM-CPL line), that was almost ineffective if administered alone (CPL line).

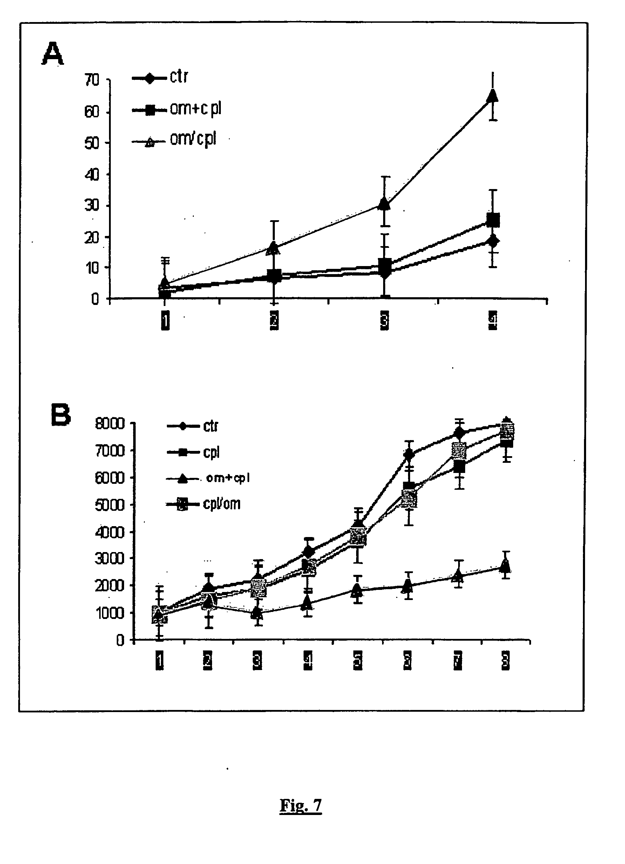

**Figure 7: Treatment strategies**

(A) Dose response curve to three logarithmic dilutions of cisplatin administered contemporary (om/cpl) or after (om+cpl) omeprazole treatment of a representative human melanoma cell line derived from a primary lesion. The results (mean from three independent experiments) showed that only pre-treatment of melanoma cells with omeprazole was able to revert cisplatin resistance. The graph shows the percentage of dead cells in function of drug concentration (X axis: 0 $\mu$M, 0,5 $\mu$M, 5 $\mu$M, 50 $\mu$M). Error bars = standard deviation.

**(B)** The Figure shows the mean results of three representative *in vivo* experiment on CB.17 *scid/scid* mice engrafted with a human melanoma cell line, derived from a primary lesion. Mice engrafted with tumour cells were treated with cisplatin alone (cpl), after omeprazole pre-treatment (om+cpl line), at the same time of omeprazole treatment (om/cpl line), or left untreated (ctr). Tumour weight (mg) is represented as a function of time. The straightforward effect of omeprazole pre-treatment in reverting cisplatin resistance of human melanoma cells engrafted in SCID mice is noteworthy, while cisplatin/omeprazole contemporaneous treatment did not induce any inhibition drug resistance. Error bars = standard deviation.

The present invention will now be further illustrated with respect to the accompanying, non-limiting Examples.

## EXAMPLE 1

## IN VITRO EXPERIMENTS WITH OMEPRAZOLE

## MATERIALS AND METHODS

### *In vitro* Experiments

**Drugs:** Omeprazole (Astra-Zeneca), esomeprazole (Astra-Zeneca), pantoprazole (Sigma Tau), lansoprazole (Pharmacia, Sweden) and rabeprazole (Janssen) sodium salts were resuspended at 1 mg/ml in PBS1X as a stock solution and stored at -20°C.
**Tumour cells:** Human tumour cells (24 melanoma cell lines, 2 colon adenocarcinoma cell lines and 2 breast cancer cell lines) obtained from a primary tumour were cultured in buffered (with bicarbonate) or not buffered (without bicarbonate) RPMI 1640 medium enriched with 10% foetal bovine serum and antibiotics in a humidified 5% $CO_2$ and 95% air atmosphere. Tumour cells were kindly supplied by Istituto Nazionale per la cura die Tumori, Milan, Italy.
**Dose response curves:** Tumour cells, growing in suspension, were plated at $1.5 \times 10^5$ /ml, in 24 wells cell culture plate (Costar). Tumour cells growing in adherence were plated at $3 \times 10^4$ cells/well in 24 wells cell culture plate. Each drug was tested for cytotoxicity on each cell type using 4 logarithmic dilutions, as shown in the Figures. Each dilution was tested at least in triplicate in each experiment.
**Cytotoxicity assay:** Cytotoxicity was evaluated using the Trypan blue exclusion method after treatment with each chemotherapeutic drug. Briefly, after treatment, cells growing in suspension were collected, centrifuged and resuspended in PBS1X. Alternatively, cells growing in adherence were collected, pooling both adherent (live) after trypsinisation and in suspension (assumed dead) cells. Cells were thus centrifuged (10 minutes at 1500 rpm) and resuspended in PBS1X. An aliquot of the cell suspension was diluted 1:1 (v/v) with 0.4% trypan blue. After 5 minutes, cells were loaded on a haemocytometer (Neubauer) and both live (not stained) and dead (blue stained) cells were counted under a light microscope. Cell viability was assessed calculating the percentage of dead cells using the formula:

$$\% \text{ dead cells} = (\text{no. dead cells}/\text{no. dead cells} + \text{ no. live cells}) \times 100.$$

**Live/Dead Viability/Cytotoxicity Assay ®.** This assay (Molecular Probes, OR, USA) provides a two colour fluorescence cell viability assay that is based on the simultaneous determination of live and dead cells with two probes (Calcein AM and Ethidium homodimer 1) that measure two recognised parameters of cell viability - intracellular esterase activity and plasma membrane integrity. Live cells were distinguished by the presence of ubiquitous intracellular esterase activity, determined by the enzymatic conversion of the virtually non-fluorescent cell permeant Calcein AM to the intensely fluorescent calcein (ex/em 495nm/515nm), which is retained in the cell. Conversely, Ethidium homodimer 1 (EthD-1) enters damaged membranes and undergoes a 40-fold enhancement of fluorescence upon binding to nucleic acids, thereby producing a bright red fluorescence in dead cells (ex/em 495nm/595nm). EthD-1 is excluded by the intact plasma membrane of live cells. According to the manufacturer's instructions, the optimal dye concentrations for the cell types used in this study were determined, in order to achieve a distinct labelling of dead and live cells, thus permitting an accurate quantitation of cytotoxic effects. After treatment, cells growing in suspensions were collected, centrifuged, and resuspended in PBS1X. Alternatively, cells growing in adherence were collected pooling both adherent (live) after trypsinisation and in suspension (presumably dead) cells. Cells were thus centrifuged (10 minutes at 1500 rpm) and resuspended in PBS1X. Cells were thus treated with Calcein AM and EthD -1 at the final concentration of 0,1 $\mu$M and 1$\mu$M, respectively, and left at room temperature for 30 minutes. After this incubation period, cells were washed once in PBS 1X and resuspended again in PBS1X. The samples were analysed with a FACScan cytometer (Becton Dickinson) equipped with a 488 argon laser. At least 20,000 events were acquired. Data were recorded and statistically analysed by a Macintosh computer using CellQuest Software. Calculation of fluorescence (expressed as median value) was carried out after conversion of logarithmically amplified signals into values on a linear scale and the statistical significance was calculated by using the parametric Kolmogorov-Smirnov (K/S) test. Statistical analysis of apoptosis data was performed using Student's *t*-test. All data reported are the mean of at least 4 separate experiments $\pm$ standard deviation (S.D.). Only p values of less than 0.01 were considered as significant. The resulting bivariate frequency distribution showed the clear separation between the green fluorescent (530 nm) live cell population and the red fluorescent (585 nm) dead-cell population (whenever present).

**Statistical analysis:** Statistical comparisons were carried out using Student's *t*-test for unpaired two-tailed comparisons. A p value of less than 0.05 was considered significant.

## RESULTS

### Omeprazole is cytotoxic for tumour cells *in vitro*

The aim of the first of the experiments was to establish whether inhibition of VH[+] ATPases through treatment with omeprazole and the other PPIs would be cytotoxic for tumour cells. The cytotoxic effect of omeprazole was tested on 24 human melanoma, 2 human colon adenocarcinoma and 2 human breast cancer cell lines deriving from primary lesions. These cells were all able to grow, without any affects on cell cycle or viability, in slightly acidic culture medium, represented by RPMI 1640 medium not supplemented with bicarbonate. This was tested, as previous data had shown that the tumour microenvironment is slightly acidic by comparison with respect to normal tissues.

In order to verify that tumour cells are susceptible to omeprazole, we used experimental conditions represented by cells grown in slightly acidic media, on which we performed an omeprazole dose response curve. As a control, the same experiment was performed culturing cells in buffered medium (pH 7.2). In Figure 1, the results on one representative human melanoma cell line are shown. A dose response curve of omeprazole was obtained treating cells with five logarithmic dilutions of the drug in buffered neutral or non buffered acidic medium (Figure 1). The results showed that: (i) omeprazole alone exhibited no apparent cytotoxic effect on the cells tested in neutral pH medium but (ii) the presence of omeprazole in the acidic medium markedly exerted a cytotoxic effect on tumour cells, in a dose-dependent manner. The experiments performed on other tumour cell lines and using the other PPIs gave comparable results (data not shown). The results were fully verified by the Live/Dead viability/cytotoxicity assay.

## EXAMPLE 2

## IN VIVO EXPERIMENT WITH OMEPRAZOLE

## MATERIALS AND METHODS

### In vivo

**Animals:** CB.17 SCID/SCID female mice (Harlan, Italy) were used at 4-5 weeks of age and were kept under specific pathogen-free conditions. SCID mice were housed in microisolator cages, and all food, water and bedding were autoclaved prior to use.

**Tumour cells:** Human tumour cells (melanoma, colon adenocarcinoma) obtained from both primary lesions were cultured in RPMI 1640 supplemented with 10% FCS, in humidified 5% $CO_2$ and 95% air atmosphere.

**Transplantation and growth of human tumours in SCID mice:** Each mouse was injected subcutaneously (s.c.) into the right flank with $3 \times 10^6$ cells resuspended in 0.2 ml RPMI 1640 supplemented with 10% FCS. After transplantation, the size of each tumour was measured using callipers. The tumour weight was estimated according to Geran *et al.* (1972) using the formula:

$$\text{Tumour weight (mg)} = \text{length (mm)} \times \text{width}^2 \text{ (mm)} / 2.$$

**Mice treatment:** Omeprazole (Astra-Zeneca, Italy) and pantoprazole (Sigma Tau) were administered at a dose of 75 mg/kg by gavage as previously described (Watson and Smith, 2001), as a suspension in PBS1X. Lansoprazole (Pharmacia, Sweden) and rabeprazole (Janssen) were administered at a dose of 25 mg/kg by gavage as previously described (Watson and Smith, 2001), as a suspension in PBS1X.

**Other:** unless otherwise specified, other parameters are as set out in Example 1, above.

## RESULTS

**The effects of Omeprazole on human tumours growth as assessed *in vivo* in SCID mice engrafted with human tumour cells.**

The *in vitro* experiments of Example 1 showed a straightforward cytotoxic effect of omeprazole on human tumour cell lines under slightly acidic conditions. Thus, efficacy was next tested in *in vivo* systems.

To this end, we tested the effects of omeprazole and its analogues on a human/mouse model system represented by CB.17 *scid/scid* mice engrafted by subcutaneous (s.c.) injection with human melanoma cells. This model has proven useful to test *in vivo* the efficacy of various anti-tumour therapies against human tumours, using either local or systemic treatment strategies (Lozupone *et al.,* 2000, 2003, 2004 in press). Mice engrafted with human tumour cells were treated with omeprazole by gavage. The effects of the treatments were measured in terms of tumour growth at different time points. The results showed that repeated omeprazole treatments markedly reduced tumour growth (Figure 2). The results obtained treating mice with omeprazole analogues are fully comparable to that shown with omeprazole (not shown). Notably, histological examination of the human tumours after the experiments were stopped showed that, in the tumours from the omeprazole-treated mice, the tumour mass was occupied by a huge necrotic area that mostly accounted for the tumour size (not shown), suggesting that the cytotoxic effect was considerably greater than that quantified by the *in vivo* tumour size measurements.

## EXAMPLE 3

## IN VITRO DRUGS AND PPIs

## MATERIALS AND METHODS

### In vitro experiments

Drugs: The PPIs were as in Example 1, above. Cisplatin (Aventis, France) was resuspended in PBS1X at a stock concentration of 1 mg/ml, and stored at-20°C. Both stock solutions were thawed immediately before use and not frozen again. 5-Fluorouracil (Teva Pharma, Holland) was supplied in the form of a solution at a concentration of 50 mg/ml, and was stored at r.t. as indicated by the supplier. Vinblastine sulphate (Eli Lilly, Paris, France) was resuspended in a solution of EtOH/Distilled water 1:1000, at a concentration of 0,1 mg/ml, thus obtaining a stock solution stored at 4°C and used in 3 days after resuspension.

**Tumour cells:** Human tumour cells were as in Example 1, above.

The MDR variant (CEM-VBL100) of CCRF-CEM (CEM) cells were obtained by exposing the parental drug-sensitive human T-lymphoblastoma cell line to increasing sublethal concentrations of vinblastine sulphate (VBL) up to 100 ng/mL (Eli Lilly, Paris, France). All the cells used in this study were cultured in RPMI 1640 medium enriched with 10% foetal bovine serum and antibiotics (basic medium, BM) in a humidified 5% $CO_2$ and 95% air atmosphere.

The MDR variant (MCF7/DX) of MCF7 was obtained by exposing the parental drug-sensitive human T-lymphoblastoma cell line to increasing sublethal concentrations of doxorubicin (DX) up to 200 ng/mL (Pharmacia & Upjohn, Italy).

**Dose response curves:** Tumour cells growing in suspension were plated at $1.5\times10^5$ /ml, in 24 wells cell culture plate (Costar). Tumour cells growing in adherence were plated at $3\times10^4$ cells/well in 24 wells cell culture plate. Each drug was tested for cytotoxicity on each cell type using 3 to 5 logarithmic dilutions, as shown in the Figures. Each dilution was tested at least in triplicate in each experiment.

**Other:** unless otherwise specified, other parameters are as set out in Example 1, above.

**RESULTS**

**Omeprazole enhanced susceptibility of human tumour cells to cisplatin**

The aim of the first of experiments was to verify that the inhibition of VH+ ATPases through treatment with omeprazole and the other PPIs could reverse multidrug resistance of tumour cells. Cisplatin, having the chemical characteristics of a weak base, due to the presence of two amine groups in its molecule, was selected to test the activity of omeprazole as a revertant of weak basic drugs resistance of tumour cells. It had been previously shown that cisplatin resistant tumour cells display higher cellular pH (and lower extra-cellular pH), together with an enhanced expression of vacuolar proton pump genes (Murakami *et al.,* 2002). The effect of omeprazole on cellular resistance to cisplatin was tested on 24 human melanoma, 2 colon-human adenocarcinoma and 2 breast cancer cell lines deriving from primary lesions, and selected for their resistance to cisplatin. In Figure 3, the results on three representative melanoma cell lines are shown. A dose response curve for cisplatin was obtained by treating cells with three logarithmic dilutions of the drug alone, or in the presence of omeprazole. The results showed that: (i) omeprazole alone did not exhibit any cytotoxic effect on the cells tested, under these conditions, and (ii) the presence of omeprazole in the culture medium markedly enhanced susceptibility to cisplatin in all the human melanoma cells tested. The experiments performed on the other tumour cell lines and using the other PPIs gave comparable results (data not shown).

**Omeprazole enhanced susceptibility of human tumour cells to 5-fluorouracil**

The possibility that PPIs act on the physiological barriers raised by tumour cells to resist to any attack, altering intra- and/or extra-cellular pH, was next investigated. In this experiment, we attempted to verify that this mechanism was effective also in reverting tumour resistance to chemotherapeutic drugs, other than the weak bases. Applying the same experimental design described before, we tested the efficacy of omeprazole on reverting primary tumour cells resistance to 5-fluorouracil (5-FU). This drug is a derivative of uracil and an analogue of folic acid, and exhibits weak acid characteristics. The effect of omeprazole on cellular resistance to 5-FU was tested on 24 human melanoma, 2 human colon adenocarcinoma and 2 human breast cancer cell lines deriving from primary lesions, and chosen for their resistance to 5FU. Figure 4 shows the results of three representative experiments. A dose response curve was obtained treating cells with 5 different logarithmic dilutions of 5-FU alone, or in the presence of omeprazole and its analogues (not shown). The results clearly showed that 5-fluorouracil sensitivity was restored by pretreating cells with omeprazole. Comparable results were obtained, using the other PPIs, as well as other tumour cell lines (not shown).

**The effects of Omeprazole on human cell lines *in vitro* selected for MDR**

In order to verify whether alteration of cellular pH could be a mechanism responsible for multidrug resistance as a basal physiological barrier, we tested the effects of omeprazole in cells *in vitro* selected for MDR phenotype and expressing P-glycoprotein as the sole transporter responsible for drug efflux. Particularly, we tested the effects of omeprazole on CEM-VBL100 cells (Figure 5), obtained by selection of the parental human lymphoblastoid CD4+Tcell line CCRF-CEM in a medium containing increasing concentrations of vinblastine sulphate up to 100 ng/ml. CEM-VBL 100 cells express P-glycoprotein and display resistance to100 ng/ml vinblastine and to other related drugs. Figure 5 shows the results of three representative experiments. A dose response curve was obtained by treating CEM-VBL100 cells with 5 different logarithmic dilutions of vinblastine-sulphate alone or in the presence of omeprazole and its analogues (not shown). The results clearly showed that vinblastine-sulphate sensitivity was restored pretreating cells with omeprazole. Comparable results were obtained by using the other PPIs (not shown), as well as performing the same experiments on the MCF7-DX cell line, obtained by selection of MCF7 parental human breast cancer cell line (not shown).

## EXAMPLE 4

## IN VIVO DRUGS AND PPIs

## MATERIALS AND METHODS

### In vivo experiments

**Cisplatin** (Aventis, France) was administered intraperitoneally (i.p.) at a dose of 5 mg/kg (Son and Huang 1994).
**Other:** unless otherwise specified, other parameters are as set out in Examples 1 and 2, above.
**The effects of Omeprazole on sensitivity of human tumours of chemotherapeutic agents as assessed *in vivo* in SCID mice engrafted with human tumour cells.**

## RESULTS

The *in vitro* experiments had shown a straightforward effect of omeprazole treatment in restoring sensitivity to cytotoxic drugs in human tumour cell lines. However, we needed to test efficacy in *in vivo* systems. To this purpose, we tested the effects of omeprazole and its analogues on a human/mouse model system represented by CB.17 *scid/scid* mice engrafted by subcutaneous (s.c.) injection human melanoma cells. This model has proven useful to test *in vivo* the efficacy of various anti-tumour therapies against human tumours, using either local or systemic treatment strategies (Lozupone 2000, 2002, in press). Mice engrafted with human tumour cells were pre-treated with omeprazole (by gavage) and i.p. with a single dose of cisplatin. The effect of the treatments was measured in terms of tumour growth at different time points. The results (Figure 6) showed that omeprazole pre-treatment markedly increased tumour sensitivity to cisplatin, while cisplatin *per se* did not show a significant effect on tumour growth. The results obtained pretreating mice with omeprazole analogues are fully comparable to that shown with omeprazole (not shown). Once again, histological examination of the human tumours after stopping the experiments showed that, in the tumours from the omeprazole/cisplatin-treated mice, the tumour mass was occupied by a huge necrotic area that mostly accounted for the tumour size (not shown), suggesting that the cytotoxic effect was greater than that quantified by the *in vivo* tumour size measurements.

### Treatment strategies

Treatment with anti-tumour drugs, such as cisplatin, might enhance the activity of V-H$^+$-ATPase (Murakami T et al 2001). Thus contemporaneous treatment with PPIs and cisplatin, or pre-treatment with cisplatin, might reduce the revertant effect of PPI. Accordingly, we performed experiments in order to assess the possible ineffective combination treatments accounting for a failure of PPI revertant effect. *In vitro* experiments clearly showed that both cisplatin pre-treatment (not shown) and omeprazole-cisplatin co-treatment did not lead to any measurable effect on the viability of human melanoma cells (Fig. 7A). Consistently, *in vivo* co-treatment with cisplatin and omeprazole of melanoma-bearing SCID mice did not show any significant effect on tumour growth (Fig. 7B).

## References

1. Altan N, Chen Y, Schindler M and Sanford SM. Defective acidification in human breast tumor cells and implications for chemotherapy. J Exp Med 1998; 187(10):1583-1598.
2. Andreola G, Rivoltini L, Castelli C, Huber V, Perego P, Deho P, Squarcina P, Accomero P., Lozupone F, Lugini L, Stringaro A, Molinari A, Arancia G, Gentile M, Parmiani G and Fais S. Induction of Lymphocytes apoptosis by tumor cell secretion of FasL-bearing microvesicles. J. Exp. Med. 2002;10:1303-1316.
3. Beck WT. The cell biology of multiple drug resistance. Biochem Pharmacol. 1987;36:2879-87
4. Bour-Dill C, Gramain MP, Merlin JL, Marchal S and Guillemin F. Determination of intracellular organelles implicated in daunorubicin cytoplasmic sequestration in multidrug-resistant MCF-7 cells using fluorescence microscopy image analysis. Cytometry. 2000;39(1):16-25.

5. Cleary I, Doherty G, Moran E and Clynes M. The multidrug-resistant human lung tumour cell line, DLKP-A10, expresses novel drug accumulation and sequestration systems. Biochem Pharmacol. 1997;53(10):1493-502.

6. Geran RI, Greenberg NH, Macdonald MM, Shumacher AM and Abbot BJ. Protocols for screening chmical agents and natural products against animal tumors and natural other biological systems. Cancer Chemother. Rep. 1972;3: 1-88.

7. Gottesman MM and Pastan I. Biochemistry of multidrug resistance mediated by the multidrug transporter. Annu. Rev. Biochem. 1993; 62:385-427.

8. Gottlieb RA, Giesing HA, Zhu JY, Engler RL, Babior BM. Cell acidification in apoptosis: granulocyte colony-stimulating factor delays programmed cell death in neutrophils by up-regulating the vacuolar H+-ATPase. Proc Natl Acad Sci USA 1995;92:5965- 8.

9. Graber ML and Devine P. Omeprazole and SCH 28080 inhibit acid secretion by the turtle urinary bladder. Ren. Physiol. Biochem. 1993;16:257-267.

10. Helmlinger G, Yuan F, Dellian M, Jain RK. Interstitial pH and pO2 gradients in solid tumors in vivo: high-resolution measurements reveal a lack of correlation. Nat Med 1997;3:177- 82.

11. Horn J. The proton-pump inhibitors: similarities and differences. Clin. Ther. 2000;22(3):266-280.

12. Hurwitz SJ, Terashima M, Mizunuma N and Slapak CA. Vesicular anthracycline accumulation in doxorubicin-selected U937 cells: participation of lysosomes. Blood. 1997;89(19):3745-3754.

13. Izumi H, Torigoe T, Ishiguchu H, Uramoto H, Yoshida Y, Tanabe M, Ise T, Murakami T, Yoshida T, Nomoto M and Kohno K. Cellular pH regulators: potentially promising molecular targets for cancer chemotherapy. Cancer Treat. Rev.2003;29:541-549.

14. Larsen AK, Escargueil AE and Skladanowski A. Resistance mechanisms associated with altered intracellular distribution of anticancer agents. Pharmacol. Therap. 2000; 85:217-229.

15. Larsson H, Mattson H, Sundell G and Carlsson E. Animal pharmacodynamics of omeprasole. A survey of its pharmacological properties in vivo. Scand. J. Gastroenterol. 1985;20(suppl.108):23-35.

16. Lozupone F, Luciani F, Lugini L, Federici F, Ramoni C, Rivoltini L, Parmiani G, Belardelli F, Rivera P, Marcenaro S, Moretta L and Fais S. Effect of human NK and gamma/delta T cells on the growth of human autologous melanoma xenografts in SCID mice. Cancer Res. 2004 64: 378-385.

17. Lozupone F, Luciani F, Venditti M, Rivoltini L, Pupa S, Parmiani G, Belardelli F and Fais S. Murine granulocytes control human tumor growth in SCID mice. Int J Cancer. 2000;87:569-573.

18. Lozupone F, Pende D, Bugio VL, Castelli C, Spada M, Venditti M, Luciani F, Lozupone, F., Rivoltini, L., Lugini, L., Cova, A., Squarcina, P., Parmiani, G., Belardelli, F. and FAIS S. Adoptive transfer of an anti-MART-127-35-specific CD8+ T cell clone leads to immunoselection of human melanoma antigen-loss variants in SCID mice. Eur J. Immunol. 2003;33:556-566.

19. Lugini L, Lozupone F, Matarrese P, Funaro C, Luciani F, Malorni W, Rivoltini L, Castelli C, Tinari A, Piris A, Parmiani G and Fais S. Potent Phagocytic Activity Discriminates Metastatic and Primary Human Malignant Melanomas: A Key Role of Ezrin. Lab. Inv. 2003; 83:1555-1567.

20. Mahoney BP, Raghunand N, Bagget B and Gillies RJ. Tumor acidity, ion trapping and chemotherapeutics I. Acid pH affects the distribution of chemotherapeutic agents in vitro. Biochem. Pharmacol. 2003;66:1207-1218.

21. Marquardt D and Center MS. Involvement of vacuolar H+-adenosine triphosphatase activity in multidrug resistance in HL60 cells. J. Natl. Cncer Inst. 1991;83:1098-1102.

22. Martinez-Zaguilan R, Lynch RM, Martinez GM and Gillies RJ. Vacuolar-type H+ATPases are functionally expressed in the plasma membranes of human tumor cells. Am. J. Physiol. 1993;265:C1015-C1029.

23. Martinez-Zaguilan R, Martinez GM, Gomez A, Hendrix MJ, Gillies RJ. Distinct regulation of pHin and [Ca21]in in human melanoma cells with different metastatic potential. J Cell Physiol 1998;176:196 -205.

24. Martinez-Zaguilan R, Raghunand N, Lynch RM, Bellamy W, Martinez GM, Rojas B, Smith D, Dalton WS and Gillies RJ. pH and drug resistance. I. Functional expression of Plasmalemmal V-type H+-ATPase in drug resistant human breast carcinoma cell lines. Biochem. Pharmacol. 1999;57:1037-1046.

25. Mizunashi K, Furukawa Y, Katano K and Abe K. Effect of omeprazole, an inhibitor of H+,K(+)-ATPase, on bone resorption in humans. Calcif. Tissue Int. 1993;53:21-25.

26. Molinari A, Calcabrini A, Meschini S, Stringaro A, Crateri P, Toccacieli L, Marra M, Colone M, Cianfriglia M, Arancia G. Subcellular detection and localization of the drug transporter P-glycoprotein in cultured tumor cells. Curr Protein Pept Sci. 2002;3:653-670.

27. Molinari A, Calcabrini A, Meschini S, Stringaro A, Del Bufalo D, Cianfriglia M, Arancia G. Detection of P-glycoprotein in the Golgi apparatus of drug-untreated human melanoma cells. Int J Cancer. 1998;75:885-893.

28. Molinari A, Toccacieli L, Calcabrini A, Diociaiuti M, Cianfriglia M, Arancia G. Induction of P-glycoprotein expression on the plasma membrane of human melanoma cells. Anticancer Res. 2000;20:2691-2696.

29. Montcourrier P, Mangeat PH, Valembois C, Salazar G, Sahunquet A, Duperray C and Rochefort H. Characterization of very acidic phagosomes in breast cancer cells and their association with invasion. J. Cell Sci. 1994;107:

2381-2391.

30. Moriyama Y. Membrane energization by proton pumps is important for compartmentalization of drugs and toxins: a new type of active transport. J Exp Biol. 1996;199:1447-54.

31. Murakami T, Shibuya I, Ise T, Chen ZS, Akiyama S, Nakagawa M, Izumi H, Nakamura T, Matsuo K, Yamada Y and Kohno K. Elevated expression of vacuolar proton pump genes and cellular PH in cisplatin resistance. Int J Cancer. 2001;93:869-74.

32. Nishi T and Forgac M. The vacuolar (H+)-ATPases - Nature'smost versatile proton pumps. Nat. Rev. Mol. Cell Biol. 2002;3:94-103.

33. Ouar Z, Bens M, Vignes C, Paulais M, Pringel C, Fleury J, Cluzeaud F, Lacave R and Vandewalle A. Inhibitors of vacuolar H+-ATPase impair the preferential accumulation of daunomycin in lysosomes and reverse the resistance to anthracyclines in drug-resistant renal epithelial cells. Biochem J. 2003;370(1):185-193.

34. Ouar Z, Lacave R, Bens M and Vandewalle A. Mechanisms of altered sequestration and efflux of chemotherapeutic drugs by multidrug resistant cells. Cell Biol. Toxicol. 1999;15:91-100.

35. Pauli-Magnus C, Rekersbrink S, Klotz U and Fromm MF. Interaction of omeprazole, lansoprazole and pantoprazole with P-glycoprotein. Arch Pharmacol. 2001;364:551-557.

36. Perona R, Serrano R. Increased pH and tumorigenicity of fibroblasts expressing a yeast proton pump. Nature 1988;334:438-40.

37. Puscas I, Coltau M, Baican M and Domuta G. Omeprazole Has a Dual Mechanism of Action: It Inhibits BothH+K+ATPase and Gastric Mucosa Carbonic Anhydrase Enzyme in Humans (In Vitro and In Vivo Experiments). J. Pharmacol. Exp. Ther. 1999;290(2):530-534.

38. Raghunand N, He X, van Sluis R, Mahoney BP, Bagget B, Tayloe CW, Paine-Murrieta G, Roe D, Bhujwalla ZM and Gillies RJ. Enhancement of chemotherapy by manipulation of tumor pH. Br. J. Cancer. 1999;80(7):1005-1011.

39. Raghunand N, Mahoney BP and Gillies RJ. Tumor acidity, ion trapping and chemotherapeutics II. PH-dependent partition coefficients predict importance of ion trapping on pharmacokinetics of weakly basic therapeutic agents. Biochem. Pharmacol. 2003;66:1219-1229.

40. Raghunand N, Martinez-Zaguilan R, Wright SH and Gillies RJ. pH and drug resistance. II. Turnover of acidic vesicles and resistance to weakly basic chemotherapeutiv drugs. Biochem. Pharmacol. 1999;57:1047-1058.

41. Sabolic I, Brown D, Verbavatz JM and Kleinman J. H(+)-ATPases of renal cortical and medullary endosomes are differentially sensitive to Sch-28080 and omeprazole. Am. J. Physiol. 1994;266: F868-877.

42. Schindler M, Grabski S, Hoff E, Simon SM. Defective pH regulation of acidic compartments in human breast cancer cells (MCF-7) is normalized in adriamycin-resistant cells (MCF-7adr). Biochemistry. 1996;35(9):2811-7.

43. Schlappack OK, Zimmermann A, Hill RP. Glucose starvation and acidosis: effect on experimental metastatic potential, DNA content and MTX resistance of murine tumour cells. Br J Cancer 1991;64:663-70.

44. Simon S, Roy D and Schindler M. Intracellular pH and the control of multidrug resistance. Proc. Natl. Acad. USA. 1994; 91:1128-1132.

45. Son K and Huang L. Exposure of human ovarian carcinoma to cisplatin transiently sensitizes the tumor cells for liposome mediated gene transfer. Proc. Natl. Acad. USA. 1994;91:12669-12672.

46. Tannock IF and Rotin D. Acid pH in tumors and its potential for therapeutic exploitation. Cancer Res. 1989;49 (16):4373-4384.

47. Torigoe T, Izumi H, Ishiguchi H, Uramoto H, Murakami T, Ise T, Yoshida Y, Tanabe M, Nomoto M, Itoh H and Kohno K. Enhanced expression of the human vacuolar H+-ATPase c subunit gene (ATP6L) in response to anticancer agents. J. Biol. Chem. 2002;277(39):36534-36543.

48. Vaananen HK, Karhukorpi EK, Sunquist K, Wallmark B, Roinine I, Hentune T, Tuukkanen J and Lakkakorpi P. Evidence of the presence of H+-ATPase types in the ruffled borders of osteoclasts. J. Cell Biol. 1990;111:1305-1311.

49. Wallmark B, Larsson H and Humble L. The Relationship between Gastric Acid Secretion and Gastric H+,K+-ATPase Activity. J. Biol. Chem. 1985;260(25):13681-13684.

50. Wallmark B, Lorentzon P and Larsson H. The mechanism of action of omeprazole - a survey of its inhibitory action in vivo. Scand. J. Gastroenterol. 1985;20(suppl.108):37-51.

51. Watson SA and Smith AM. Hypergastrinaemia promotes adenomaprogression in the APCMin7/+ mouse model of familial adenomatous polyposis. Cancer Res. 2001;61:625-631.

## Claims

1. A 2-pyridyl methylsulphinyl benzimidazole proton pump inhibitor (PPI) for use in the systemic, therapeutic treatment of a solid tumour, by oral administration.

2. A PPI for use according to claim 1, wherein the tumour is metastatic.

**3.** A PPI for use according to claim 1 or 2, wherein the proton pump inhibitor is selected from omeprazole, lansoprazole, pantoprazole, esomeprazole, rabeprazole, and mixtures thereof.

**4.** A PPI for use according to any preceding claim, wherein the treatment is of a patient who has been treated with an antacid.

**5.** A PPI for use according to claim 4 wherein the antacid is to be administered prior to the proton pump inhibitor.

**6.** A PPI for use according to any of claims 4 to 5 wherein the antacid is calcium carbonate.

**7.** A PPI for use according to any of claims 4 to 5, wherein the antacid is an $H_2$-receptor antagonist.

**8.** A PPI for use according to any one of the preceding claims, wherein the proton pump inhibitor is to be administered prior to at least one further drug indicated against said the tumour.

**9.** A PPI for use according to claim 8, wherein the period prior to administration of the further drug is between 30 minutes and 3 days.

**10.** A PPI for use according to any of claims 8 to 9, wherein the further drug is selected from: vinka alkaloids; taxanes; anthracyclines; anthracenes; epipodophyllotoxins; camptothecins; heavy metal oxyanions; actinomycin d; mitomycin c; methotrexate; trimetrexate; amsacrine; imitinib; and melphalan; 5-fluorouracil; and cisplatin.

**11.** A PPI for use according to any of claims 8 to 10, wherein the disease condition is resistant to the further drug.

**12.** A PPI for use according to any preceding claim, wherein the proton pump inhibitor is omeprazole.

**Patentansprüche**

**1.** 2-Pyridylmethylsulfinylbenzimidazol-Protonenpumpenhemmer (PPH) zur Verwendung bei der systemischen therapeutischen Behandlung eines soliden Tumors durch orale Verabreichung.

**2.** PPH zur Verwendung nach Anspruch 1, wobei der Tumor metastatisch ist.

**3.** PPH zur Verwendung nach Anspruch 1 oder 2, wobei der Protonenpumpenhemmer unter Omeprazol, Lansoprazol, Pantoprazol, Esomeprazol, Rabeprazol und Gemischen davon ausgewählt ist.

**4.** PPH zur Verwendung nach einem der vorstehenden Ansprüche, wobei die Behandlung an einem Patienten durchgeführt wird, der mit einem Antazidum behandelt worden ist.

**5.** PPH zur Verwendung nach Anspruch 4, wobei das Antazidum vor dem Protonenpumpenhemmer zu verabreichen ist.

**6.** PPH zur Verwendung nach einem der Ansprüche 4 bis 5, wobei das Antazidum Calciumcarbonat ist.

**7.** PPH zur Verwendung nach einem der Ansprüche 4 bis 5, wobei das Antazidum ein $H_2$-Rezeptor-Antagonist ist.

**8.** PPH zur Verwendung nach einem der vorstehenden Ansprüche, wobei der Protonenpumpenhemmer vor mindestens einem weiteren, gegen den Tumor indizierten Medikament zu verabreichen ist.

**9.** PPH zur Verwendung nach Anspruch 8, wobei die Zeitspanne vor der Verabreichung des weiteren Medikaments zwischen 30 Minuten und 3 Tagen liegt.

**10.** PPH zur Verwendung nach einem der Ansprüche 8 bis 9, wobei das weitere Medikament unter Vincaalkaloiden; Taxanen; Anthracyclinen; Anthracenen; Epipodophyllotoxinen, Camptothecinen, Schwermetall-Oxyanionen; Actinomycin d; Mitomycin c; Methotrexat; Trimetrexat; Amsacrin; Imitinib und Melphalan; 5-Flururacil und Cisplatin ausgewählt ist.

**11.** PPH zur Verwendung nach einem der Ansprüche 8 bis 10, wobei der Krankheitszustand resistent gegen das weitere Medikament ist.

**12.** PPH zur Verwendung nach einem der vorstehenden Ansprüche, wobei der Protonenpumpenhemmer Omeprazol ist.

**Revendications**

**1.** Inhibiteur de pompe à protons (PPI) de 2-pyridyl-méthylsulfinyl-benzimidazole pour une utilisation dans le traitement thérapeutique systémique d'une tumeur solide, par une administration orale.

**2.** PPI pour une utilisation selon la revendication 1, dans lequel la tumeur est métastatique.

**3.** PPI pour une utilisation selon la revendication 1 ou 2, où l'inhibiteur de pompe à protons est choisi parmi l'oméprazole, le lansoprazole, le pantoprazole, l'ésoméprazole, le rabéprazole et des mélanges de ceux-ci.

**4.** PPI pour une utilisation selon l'une quelconque des revendications précédentes, dans lequel le traitement est d'un patient qui a été traité avec un antiacide.

**5.** PPI pour une utilisation selon la revendication 4, dans lequel l'antiacide est à administrer avant l'inhibiteur de pompe à protons.

**6.** PPI pour une utilisation selon l'une quelconque des revendications 4 à 5, dans lequel l'antiacide est le carbonate de calcium.

**7.** PPI pour une utilisation selon l'une quelconque des revendications 4 à 5, dans lequel l'antiacide est un antagoniste de récepteur $H_2$.

**8.** PPI pour une utilisation selon l'une quelconque des revendications précédentes, où l'inhibiteur de pompe à protons est à administrer avant au moins un médicament supplémentaire indiqué contre ladite tumeur.

**9.** PPI pour une utilisation selon la revendication 8, dans lequel la période avant l'administration du médicament supplémentaire est entre 30 minutes et 3 jours.

**10.** PPI pour une utilisation selon l'une quelconque des revendications 8 à 9, dans lequel le médicament supplémentaire est choisi parmi: des alcaloïdes de la pervenche; des taxanes; des anthracyclines; des anthracènes; des épipodophyllotoxines; des camptothécines; des oxyanions de métaux lourds; l'actinomycine d; la mitomycine c; le méthotrexate; le trimétrexate; l'amsacrine; l'imitinib; et le melphalan; le 5-fluorouracile; et le cisplatine.

**11.** PPI pour une utilisation selon l'une quelconque des revendications 8 à 10, dans lequel l'état pathologique est résistant au médicament supplémentaire.

**12.** PPI pour une utilisation selon l'une quelconque des revendications précédentes, où l'inhibiteur de pompe à protons est l'oméprazole.

Figure 1

Figure 2

**Fig. 3**

**Fig. 4**

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003277262 B
- JP 2001286284 B
- WO 02080917 A

- EP 0567643 A
- EP 1306375 A
- WO 89103829 A


### Non-patent literature cited in the description

- **ALTAN N ; CHEN Y ; SCHINDLER M ; SANFORD SM.** Defective acidification in human breast tumor cells and implications for chemotherapy. *J Exp Med,* 1998, vol. 187 (10), 1583-1598
- **ANDREOLA G ; RIVOLTINI L ; CASTELLI C ; HUBER V ; PEREGO P ; DEHO P ; SQUARCINA P ; ACCOMERO P. ; LOZUPONE F ; LUGINI L.** Induction of Lymphocytes apoptosis by tumor cell secretion of FasL-bearing microvesicles. *J. Exp. Med.,* 2002, vol. 10, 1303-1316
- **BECK WT.** The cell biology of multiple drug resistance. *Biochem Pharmacol.,* 1987, vol. 36, 2879-87
- **BOUR-DILL C ; GRAMAIN MP ; MERLIN JL ; MARCHAL S ; GUILLEMIN F.** Determination of intracellular organelles implicated in daunorubicin cytoplasmic sequestration in multidrug-resistant MCF-7 cells using fluorescence microscopy image analysis. *Cytometry,* 2000, vol. 39 (1), 16-25
- **CLEARY I ; DOHERTY G ; MORAN E ; CLYNES M.** The multidrug-resistant human lung tumour cell line, DLKP-A10, expresses novel drug accumulation and sequestration systems. *Biochem Pharmacol.,* 1997, vol. 53 (10), 1493-502
- **GERAN RI ; GREENBERG NH ; MACDONALD MM ; SHUMACHER AM ; ABBOT BJ.** Protocols for screening chmical agents and natural products against animal tumors and natural other biological systems. *Cancer Chemother. Rep.,* 1972, vol. 3, 1-88
- **GOTTESMAN MM ; PASTAN I.** Biochemistry of multidrug resistance mediated by the multidrug transporter. *Annu. Rev. Biochem.,* 1993, vol. 62, 385-427
- **GOTTLIEB RA ; GIESING HA ; ZHU JY ; ENGLER RL ; BABIOR BM.** Cell acidification in apoptosis: granulocyte colony-stimulating factor delays programmed cell death in neutrophils by up-regulating the vacuolar H+-ATPase. *Proc Natl Acad Sci USA,* 1995, vol. 92, 5965-8

- **GRABER ML ; DEVINE P.** Omeprazole and SCH 28080 inhibit acid secretion by the turtle urinary bladder. *Ren. Physiol. Biochem.,* 1993, vol. 16, 257-267
- **HELMLINGER G ; YUAN F ; DELLIAN M ; JAIN RK.** Interstitial pH and pO2 gradients in solid tumors in vivo: high-resolution measurements reveal a lack of correlation. *Nat Med,* 1997, vol. 3, 177-82
- **HORN J.** The proton-pump inhibitors: similarities and differences. *Clin. Ther.,* 2000, vol. 22 (3), 266-280
- **HURWITZ SJ ; TERASHIMA M ; MIZUNUMA N ; SLAPAK CA.** Vesicular anthracycline accumulation in doxorubicin-selected U937 cells: participation of lysosomes. *Blood,* 1997, vol. 89 (19), 3745-3754
- **IZUMI H ; TORIGOE T ; ISHIGUCHU H ; URAMOTO H ; YOSHIDA Y ; TANABE M ; ISE T ; MURAKAMI T ; YOSHIDA T ; NOMOTO M.** Cellular pH regulators: potentially promising molecular targets for cancer chemotherapy. *Cancer Treat. Rev.,* 2003, vol. 29, 541-549
- **LARSEN AK ; ESCARGUEIL AE ; SKLADANOWSKI A.** Resistance mechanisms associated with altered intracellular distribution of anticancer agents. *Pharmacol. Therap.,* 2000, vol. 85, 217-229
- **LARSSON H ; MATTSON H ; SUNDELL G ; CARLSSON E.** Animal pharmacodynamics of omeprasole. A survey of its pharmacological properties in vivo. *Scand. J. Gastroenterol.,* 1985, vol. 20 (108), 23-35
- **LOZUPONE F ; LUCIANI F ; LUGINI L ; FEDERICI F ; RAMONI C ; RIVOLTINI L ; PARMIANI G ; BELARDELLI F ; RIVERA P ; MARCENARO S.** Effect of human NK and gamma/delta T cells on the growth of human autologous melanoma xenografts in SCID mice. *Cancer Res.,* 2004, vol. 64, 378-385

- **LOZUPONE F ; LUCIANI F ; VENDITTI M ; RIVOLT-INI L ; PUPA S ; PARMIANI G ; BELARDELLI F ; FAIS S.** Murine granulocytes control human tumor growth in SCID mice. *Int J Cancer,* 2000, vol. 87, 569-573
- **LOZUPONE F ; PENDE D ; BUGIO VL ; CASTELLI C ; SPADA M ; VENDITTI M ; LUCIANI F ; LOZUPONE, F. ; RIVOLTINI, L. ; LUGINI, L.** Adoptive transfer of an anti-MART-127-35-specific CD8+ T cell clone leads to immunoselection of human melanoma antigen-loss variants in SCID mice. *Eur J. Immunol.,* 2003, vol. 33, 556-566
- **LUGINI L ; LOZUPONE F ; MATARRESE P ; FUNARO C ; LUCIANI F ; MALORNI W ; RIVOLTINI L ; CASTELLI C ; TINARI A ; PIRIS A.** Potent Phagocytic Activity Discriminates Metastatic and Primary Human Malignant Melanomas: A Key Role of Ezrin. *Lab. Inv.,* 2003, vol. 83, 1555-1567
- **MAHONEY BP ; RAGHUNAND N ; BAGGET B ; GILLIES RJ.** Tumor acidity, ion trapping and chemotherapeutics I. Acid pH affects the distribution of chemotherapeutic agents in vitro. *Biochem. Pharmacol.,* 2003, vol. 66, 1207-1218
- **MARQUARDT D ; CENTER MS.** Involvement of vacuolar H+-adenosine triphosphatase activity in multidrug resistance in HL60 cells. *J. Natl. Cncer Inst.,* 1991, vol. 83, 1098-1102
- **MARTINEZ-ZAGUILAN R ; LYNCH RM ; MARTINEZ GM ; GILLIES RJ.** Vacuolar-type H+ATPases are functionally expressed in the plasma membranes of human tumor cells. *Am. J. Physiol.,* 1993, vol. 265, C1015-C1029
- **MARTINEZ-ZAGUILAN R ; MARTINEZ GM ; GOMEZ A ; HENDRIX MJ ; GILLIES RJ.** Distinct regulation of pHin and [Ca21]in in human melanoma cells with different metastatic potential. *J Cell Physiol,* 1998, vol. 176, 196-205
- **MARTINEZ-ZAGUILAN R ; RAGHUNAND N ; LYNCH RM ; BELLAMY W ; MARTINEZ GM ; ROJAS B ; SMITH D ; DALTON WS ; GILLIES RJ.** pH and drug resistance. I. Functional expression of Plasmalemmal V-type H+-ATPase in drug resistant human breast carcinoma cell lines. *Biochem. Pharmacol.,* 1999, vol. 57, 1037-1046
- **MIZUNASHI K ; FURUKAWA Y ; KATANO K ; ABE K.** Effect of omeprazole, an inhibitor of H+,K(+)-ATPase, on bone resorption in humans. *Calcif. Tissue Int.,* 1993, vol. 53, 21-25
- **MOLINARI A ; CALCABRINI A ; MESCHINI S ; STRINGARO A ; CRATERI P ; TOCCACIELI L ; MARRA M ; COLONE M ; CIANFRIGLIA M ; ARANCIA G.** Subcellular detection and localization of the drug transporter P-glycoprotein in cultured tumor cells. *Curr Protein Pept Sci.,* 2002, vol. 3, 653-670
- **MOLINARI A ; CALCABRINI A ; MESCHINI S ; STRINGARO A ; DEL BUFALO D ; CIANFRIGLIA M ; ARANCIA G.** Detection of P-glycoprotein in the Golgi apparatus of drug-untreated human melanoma cells. *Int J Cancer,* 1998, vol. 75, 885-893
- **MOLINARI A ; TOCCACIELI L ; CALCABRINI A ; DIOCIAIUTI M ; CIANFRIGLIA M ; ARANCIA G.** Induction of P-glycoprotein expression on the plasma membrane of human melanoma cells. *Anticancer Res.,* 2000, vol. 20, 2691-2696
- **MONTCOURRIER P ; MANGEAT PH ; VALEMBOIS C ; SALAZAR G ; SAHUNQUET A ; DUPERRAY C ; ROCHEFORT H.** Characterization of very acidic phagosomes in breast cancer cells and their association with invasion. *J. Cell Sci.,* 1994, vol. 107, 2381-2391
- **MORIYAMA Y.** Membrane energization by proton pumps is important for compartmentalization of drugs and toxins: a new type of active transport. *J Exp Biol.,* 1996, vol. 199, 1447-54
- **MURAKAMI T ; SHIBUYA I ; ISE T ; CHEN ZS ; AKIYAMA S ; NAKAGAWA M ; IZUMI H ; NAKAMURA T ; MATSUO K ; YAMADA Y.** Elevated expression of vacuolar proton pump genes and cellular PH in cisplatin resistance. *Int J Cancer,* 2001, vol. 93, 869-74
- **NISHI T ; FORGAC M.** The vacuolar (H+)-ATPases - Nature's smost versatile proton pumps. *Nat. Rev. Mol. Cell Biol.,* 2002, vol. 3, 94-103
- **OUAR Z ; BENS M ; VIGNES C ; PAULAIS M ; PRINGEL C ; FLEURY J ; CLUZEAUD F ; LACAVE R ; VANDEWALLE A.** Inhibitors of vacuolar H+-ATPase impair the preferential accumulation of daunomycin in lysosomes and reverse the resistance to anthracyclines in drug-resistant renal epithelial cells. *Biochem J.,* 2003, vol. 370 (1), 185-193
- **OUAR Z ; LACAVE R ; BENS M ; VANDEWALLE A.** Mechanisms of altered sequestration and efflux of chemotherapeutic drugs by multidrug resistant cells. *Cell Biol. Toxicol.,* 1999, vol. 15, 91-100
- **PAULI-MAGNUS C ; REKERSBRINK S ; KLOTZ U ; FROMM MF.** Interaction of omeprazole, lansoprazole and pantoprazole with P-glycoprotein. *Arch Pharmacol.,* 2001, vol. 364, 551-557
- **PERONA R ; SERRANO R.** Increased pH and tumorigenicity of fibroblasts expressing a yeast proton pump. *Nature,* 1988, vol. 334, 438-40
- **PUSCAS I ; COLTAU M ; BAICAN M ; DOMUTA G.** Omeprazole Has a Dual Mechanism of Action: It Inhibits Both H+K+ATPase and Gastric Mucosa Carbonic Anhydrase Enzyme in Humans (In Vitro and In Vivo Experiments). *J. Pharmacol. Exp. Ther.,* 1999, vol. 290 (2), 530-534

- **RAGHUNAND N ; HE X ; VAN SLUIS R ; MA-HONEY BP ; BAGGET B ; TAYLOE CW ; PAINE-MURRIETA G ; ROE D ; BHUJWALLA ZM ; GILLIES RJ.** Enhancement of chemotherapy by manipulation of tumor pH. *Br. J. Cancer,* 1999, vol. 80 (7), 1005-1011
- **RAGHUNAND N ; MAHONEY BP ; GILLIES RJ.** Tumor acidity, ion trapping and chemotherapeutics II. PH-dependent partition coefficients predict importance of ion trapping on pharmacokinetics of weakly basic therapeutic agents. *Biochem. Pharmacol.,* 2003, vol. 66, 1219-1229
- **RAGHUNAND N ; MARTINEZ-ZAGUILAN R ; WRIGHT SH ; GILLIES RJ.** pH and drug resistance. II. Turnover of acidic vesicles and resistance to weakly basic chemotherapeutiv drugs. *Biochem. Pharmacol.,* vol. 57, 1047-1058
- **SABOLIC I ; BROWN D ; VERBAVATZ JM ; KLEIN-MAN J.** H(+)-ATPases of renal cortical and medullary endosomes are differentially sensitive to Sch-28080 and omeprazole. *Am. J. Physiol.,* 1994, vol. 266, F868-877
- **SCHINDLER M ; GRABSKI S ; HOFF E ; SIMON SM.** Defective pH regulation of acidic compartments in human breast cancer cells (MCF-7) is normalized in adriamycin-resistant cells (MCF-7adr). *Biochemistry,* 1996, vol. 35 (9), 2811-7
- **SCHLAPPACK OK ; ZIMMERMANN A ; HILL RP.** Glucose starvation and acidosis: effect on experimental metastatic potential, DNA content and MTX resistance of murine tumour cells. *Br J Cancer,* 1991, vol. 64, 663-70
- **SIMON S ; ROY D ; SCHINDLER M.** Intracellular pH and the control of multidrug resistance. *Proc. Natl. Acad. USA,* 1994, vol. 91, 1128-1132
- **SON K ; HUANG L.** Exposure of human ovarian carcinoma to cisplatin transiently sensitizes the tumor cells for liposome mediated gene transfer. *Proc. Natl. Acad. USA,* 1994, vol. 91, 12669-12672
- **TANNOCK IF ; ROTIN D.** Acid pH in tumors and its potential for therapeutic exploitation. *Cancer Res.,* 1989, vol. 49 (16), 4373-4384
- **TORIGOE T ; IZUMI H ; ISHIGUCHI H ; URAMOTO H ; MURAKAMI T ; ISE T ; YOSHIDA Y ; TANABE M ; NOMOTO M ; ITOH H.** Enhanced expression of the human vacuolar H+-ATPase c subunit gene (ATP6L) in response to anticancer agents. *J. Biol. Chem.,* 2002, vol. 277 (39), 36534-36543
- **VAANANEN HK ; KARHUKORPI EK ; SUNQUIST K ; WALLMARK B ; ROININE I ; HENTUNE T ; TU-UKKANEN J ; LAKKAKORPI P.** Evidence of the presence of H+-ATPase types in the ruffled borders of osteoclasts. *J. Cell Biol.,* 1990, vol. 111, 1305-1311
- **WALLMARK B ; LARSSON H ; HUMBLE L.** The Relationship between Gastric Acid Secretion and Gastric H+,K+-ATPase Activity. *J. Biol. Chem.,* 1985, vol. 260 (25), 13681-13684
- **WALLMARK B ; LORENTZON P ; LARSSON H.** The mechanism of action of omeprazole - a survey of its inhibitory action in vivo. *Scand. J. Gastroenterol.,* 1985, vol. 20 (108), 37-51
- **WATSON SA ; SMITH AM.** Hypergastrinaemia promotes adenomaprogression in the APCMin7/+ mouse model of familial adenomatous polyposis. *Cancer Res.,* 2001, vol. 61, 625-631